# EUROPEAN PATENT SPECIFICATION

(11) **EP 0 525 163 B1**
(45) Date of publication and mention of the grant of the patent: **02.08.1995**
(21) Application number: 92905800.6
(22) Date of filing: 30.10.1991
(51) Int. Cl.: A61K 9/127

(54) **BINDING OF GROWTH HORMONE TO LIPOSOMES**
KOPPELUNG DES WACHSTUMSHORMONS AN LIPOSOMEN
LIAISON D'UNE HORMONE DE CROISSANCE AUX LIPOSOMES

(30) Priority: 14.02.1991 US 655576
(43) Date of publication of application: 03.02.1993
(73) Proprietor: BAXTER INTERNATIONAL INC., Deerfield, IL 60015 (US)
(72) Inventor: MARGALIT, Rimona, 53 318 Givataim (IL)
(74) Representative: MacGregor, Gordon
(86) International application number: US9108114
(87) International publication number: WO9214448

(56) References cited:
- WO-A-90/09782
- G. GREGORIADIS 'liposome technologie' 1984 , CRC PRESS, INC. , BOCA RATON, FLORIDA (US)
- WORLD PATENTS INDEX LATEST Week 8509, Derwent Publications Ltd., London, GB; AN 85-052381(09)
- BIOCHEMICAL AND BIOPHYSICAL RESEARCH COMMUNICATIONS vol. 160, no. 2, 28 April 1989, DULUTH ,MINN. (US) pages 732 - 736; Y. ISHII ET AL.: 'preparation of egf labeled liposomes and their uptake by hepatocytes' FLORIDA (US)

## Description

### BACKGROUND OF THE INVENTION

The present invention relates to the preparation of microscopic drug delivery systems (MDDS) utilizing drug-encapsulating bioadhesive liposomes.

Microscopic drug delivery systems (MDDS) have been developed for improved drug administration relative to administration of drugs in their free form. Drug-loaded MDDS can perform as sustained or controlled release drug depots. By providing a mutual protection of the drug and the biological environment, MDDS reduces drug degradation or inactivation. As a system for controlled release of a drug, MDDS improves drug efficacy and allows reduction in the frequency of dosing. Since the pharmacokinetics of free drug release from depots of MDDS are different than from directly-administered drug, MDDS provides an additional measure to reduce toxicity and undesirable side effects.

MDDS is divided into two basic classes: particulate systems, such as cells, microspheres, viral envelopes and liposomes; or nonparticulate systems which are macromolecules such as proteins or synthetic polymers. Liposomes have been studied as drug carriers and offer a range of advantages relative to other MDDS systems. Composed of naturally-occurring materials which are biocompatible and biodegradable, liposomes are used to encapsulate biologically active materials for a variety of purposes. Having a variety of layers, sizes, surface charges and compositions, numerous procedures for liposomal preparation and for drug encapsulation within them have been developed, some of which have been scaled up to industrial levels.

Liposomes can be designed to act as sustained release drug depots and, in certain applications, aid drug access across cell membranes. Their ability to protect encapsulated drugs and various other characteristics make liposomes a popular choice in developing MDDS, with respect to the previous practices of free drug administration.

Despite the advantages offered, utilization of drug-encapsulating liposomes does pose some difficulties. For example, liposomes as MDDS have limited targeting abilities, limited retention and stability in circulation, potential toxicity upon chronic administration and inability to extravasate. In recent years, successful attempts have been made to bind different substances to liposomes. For example, binding of chymotrypsin to liposomes has been studied as a model for binding substances to liposomal surfaces. Recognizing substances, including antibodies, glycoproteins and lectins have been bound to liposomal surfaces in an attempt to confer target specificity to the liposomes. Concentrating on systemic applications and in vivo studies, these previous efforts discuss methods of binding recognizing substances with liposomes and the effectiveness of such modified liposomes. Although the bonding of these recognizing substances to liposomes occurred, the resulting modified liposomes did not perform as hoped, particularly during in vivo studies. Other difficulties are presented when utilizing these recognizing substances. For example, antibodies can be patient specific and, therefore, add cost to the drug therapy.

It has also been previously learned that liposomes can be modified by binding epidermal growth factor (EGF) to the liposomal surface as a recognizing substance. EGF stimulates cell growth and proliferation through the interaction with the EGF receptor. The EGF receptors are distributed on the cell surface of various organs and are present in burns, wounds, and other designated targets of MDDS such as ocular, dermal and tumors. Accordingly, EGF-modified liposomes potentially offer efficiency as drug carriers to target sites expressing the EGF receptors.

EGF is a polypeptide. It has been reported that the biological activity of EGF is dependent upon the conservation of the native conformation of EGF, to which the disulfide bonds are critical. Previous work in binding EGF to liposomes has capitalized on the existence of the disulfide bonds. Specifically, EGF has been bound to the liposomal surface by the disulfide bridge linkage using a heterobifunctional crosslinking reagent, N-hydroxysuccinimidyl-3-(2-pyridyldithio) propionate. Although the resulting EGF-modified liposomes have been suggested as potential drug carriers for systemic applications, the complex chemistry of this process results in byproducts whose effect on drug delivery and toxicity are unknown.

EGF is bound covalently to discrete sites on the liposome surfaces. The number and surface density of these sites will be dictated by the liposome formulation and the liposome type. The liposomal surfaces may also have sites for noncovalent association. Covalent binding is essential as noncovalent binding might result in dissociation of EGF from the liposomes at the site of administration since the liposomes and the bioadhesive counterparts of the target site (that is, the bioadhesive matter) compete for EGF. Such dissociation would reverse the administered modified liposomes into regular, non-modified liposomes, thereby defeating the purpose of administration of the modified liposomes.

To form covalent conjugates of recognizing substances and liposomes, crosslinking reagents have been studied for effectiveness and biocompatibility. One such reagent is glutaraldehyde (GAD). Through the complex chemistry of crosslinking by GAD, linkage of the amine residues of the recognizing substance and liposomes is established. Previous efforts have studied the binding of chymotrypsin and liposomes with GAD as the crosslinking reagent.

Methodologies have been developed and crosslinking reagents have been identified to bind growth hormones, preferably epidermal growth factor (EGF), to liposomal surfaces. More specifically, crosslinking reagents have been identified which crosslink amine residues on the liposomal surfaces to the residues offered by EGF.

G. Gregonades "Liposome Technology" 1984, p.76-90 discloses a process for making modified liposomes in which liposomes containing phosphatidylethanolamine (PE) are incubated in a first step with a crosslinking agent to "activate" the liposomes, and then antimyosin antibody is added, which cross-links with the activated liposomes.

Biochemical and Biophysical Research Communications, vol. 160, no.2, 1989, p.732-736 discloses a process for making modified liposomes comprising EGF covalently linked to the surfaces of the liposomes. The liposomes contain dipalmitoylphosphatidylethanolamine, (DPPE) which is derivatized in a first step with 3-(2-pyridyldithio) proprionate (DTP) before being incorporated into liposomes to produce activated liposomes. Derivatized EGF is then added to the activated liposomes and the mixture is incubated to allow crosslinking of the EGF to the DPPE.

The present inventions provides a process for producing modified liposomes comprising the steps of:
(a) providing a reaction vessel containing a liposome component having liposome and phosphatidylethanolamine;
(b) adding a recognizing substance component of a growth factor to the reaction vessel;
(c) buffering the reaction mixture of the liposome and recognizing substance components;
(d) adding a crosslinking reagent to the buffered reaction mixture; and
(e) allowing the buffered reaction mixture with reagent to incubate for a period of time sufficient for the modified liposome to form.

The present also provides a process for covalently bonding a recognizing substance to a liposome comprising the steps of:
(a) providing a reaction vessel containing a liposome component having liposome and phosphatidylethanolamine;
(b) adding a recognizing substance component of growth factor to the reaction vessel;
(c) buffering the reaction mixture of the liposome and recognizing substance component;
(d) adding a crosslinking reagent to the buffered reaction mixture;
(e) allowing the buffered reaction mixture with reagent to ncubate for a period of time sufficient for the covalent bonding of amine residues of the recognising substance and liposome components; and
(f) separation and removal of covalently bound recognizing substance liposome components from excess unreacted and noncovalently bound materials.

Preferably, the crosslinking reagents include glutaraldehyde (GAD) and bi-functional oxirane (OXR), preferably ethylene glycol diglycidyl ether (EGDE).
By modifying regular liposomes through covalent bonding certain growth hormones to the liposomal surface, the growth hormones are recognizing substances which can be utilized as an adhesive or glue to attach the modified liposome onto a target area. These "bioadhesive" liposomes offer potential advantages as a MDDS for the administration of drugs.

### DETAILED DESCRIPTION

According to the present invention, growth hormones, preferably EGF, have been covalently bound to liposomal surfaces as a recognizing substance through the crosslinking of amine residues. Liposomes, in particular, multilamellar vesicles (MLV), microemulsified liposomes (MEL) or large unilamellar vesicles (LUVET), each containing phosphatidylethanolamine (PE), have been prepared by established procedures. Although urogasterone and EGF are recognized as biological equivalents, both purified urogasterone or EGF mouse were used as recognizing substances, with the results reported in Examples 1 and 2. In the specification and claims the term "EGF" means either urogasterone or epidermal growth factor regardless of the source. Growth hormone was linked to the PE-liposomes.

In the Examples, the EGF and lipids are assayed by traces of radioactive or fluorescent labels included in each formulation. Alternatively, the lipids are assayed by colorimetric methods. The determination of the non-labeled and the fluorescent-labeled EGF can also be performed by the Lowry procedure previously reported.

The "level of covalent binding" as reported in the Examples and Tables 1 and 2, is defined as the quantity of ligand or EGF bound to a given quantity of lipid in the final product since the most accurate quantitative measure of liposomes is in terms of lipid quantities. For a given lipid quantity, different liposome types will yield different quantities of liposome. Therefore, similar initial ratios of EGF to lipid for different liposome types should not be expected to yield the same level of binding. Another factor which would yield different results for different liposomes even under the same initial EGF to lipid ratios, is the differences in particle size, therefore in curvature, number and accessibility of PE sites on the surface of the liposome. Therefore, comparisons among liposome types should be avoided.

The effects of the increase in the EGF/lipid ratios in the presence of a crosslinking reagent are shown in Tables 1 and 2. Generally, an increase in the level of binding occurs with the increase in initial EGF/lipid ratios regardless of which crosslinking reagent is used. At the lower end of the EGF/lipid ratios, the level of covalent binding increases significantly. Beyond initial concentration ratios of 25 ng EGF/uMoles lipid, the increase of binding is less significant. Noncovalently bound product is removed as excess unreacted material and does not appear in the reported results.

### Example One

EGF is added to a PE-liposome sample and the mixture is buffered by a phosphate buffer saline solution (PBS) to pH of 7.2. Concentration ratios of EGF to lipid are shown in Table 1. Aliquots from a 25% solution of the crosslinking reagent GAD are added at a ratio of 10ul per 1 ml EGF/PE-liposome mixture. Incubation for a desired period is completed at either room temperature without stirring or at 37°C with stirring. Depending upon the liposome used, excess unreacted material was removed through centrifugation and washings, column chromatography or dialysis against PBS.

**TABLE 1**

| EGF-LIPOSOME CROSSLINKING BY GAD | | |
|---|---|---|
| LIPOSOME TYPE | ng EGF/uMOLES LIPID (a) | |
| | INITIAL | FINAL |
| MLV | 0.080 | 0.009 |
| MLV | 0.309 | 0.006 |
| MLV | 0.347 | 0.016 |
| MEL | 0.071 | 0.004 |
| MEL | 0.106 | 0.009 |
| MEL | 0.141 | 0.025 |
| LUVET | 0.016 | 0.003 |

| | | |
|---|---|---|
| (a) EGF assayed by a radioactive tracer. | | |

### Example Two

EGF is crosslinked with PE-liposome samples following the same procedure as in Example 1. Concentration ratios of labeled EGF to lipid are shown in Table 2.

**TABLE 2**

| EGF-LIPOSOME CROSSLINKING BY GAD | | |
|---|---|---|
| LIPOSOME TYPE | ng EGF/uMOLES LIPID (a) | |
| | INITIAL | FINAL |
| MLV | 0.26 | 0.07 |
| MLV | 0.78 | 0.16 |
| MLV | 1.60 | 0.21 |
| MLV | 6.00 | 0.31 |
| MLV | 24.70 | 0.35 |

| | | |
|---|---|---|
| (a) EGF assayed by a fluorescent tracer. | | |

### Example Three

Reaction mixtures of EGF and PE-liposomes were prepared as above and buffered by PBS to pH 7 or by 0.5N carbonate buffer to pH 9. Concentrations ratios of EGF to lipid are shown in Table 3. The crosslinking reagent EGDE was added in 0.2 - 1.0 ml volumes to buffered reaction mixtures of 2.5 - 3.0 ml volumes. Incubation periods were completed for 10-24 hours at 37°C with stirring. Depending upon the liposome used, excess unreacted material was removed through ultracentrifugations and washings or dialysis against PBS.

**TABLE 3**

| EGF-LIPOSOME CROSSLINKING BY EGDE | | | | |
|---|---|---|---|---|
| LIPOSOME TYPE | ng/EGF/uMOLE LIPID (a) | | pH | mg EGDE |
| | INITIAL | FINAL | | |
| MLV (b) | 0.45 | 0.0078 | 9 | 500 |
| MLV | 3.72 | 0.90 | 9 | 500 |
| MEL | 0.10 | 0.012 | 9 | 500 |
| MEL | 0.10 | 0.0098 | 9 | 1000 |
| MEL (a) | 0.12 | 0.0022 | 7 | 200 |
| MEL | 1.78 | 0.47 | 9 | 500 |

| | | | | |
|---|---|---|---|---|
| (a) EGF assayed by a radioactive tracer. | | | | |
| (b) Initial ratios were increased by decreasing lipid concentration. | | | | |

From these results, the preferred pH of 9 and quantity of crosslinking reagent of 500 mg has been determined.

The mouse EGF and human urogasterone used in the disclosed examples could be substituted with growth factors from other natural or synthetic sources.

## Claims

1. A process for producing modified liposomes comprising the steps of:
(a) providing a reaction vessel containing a liposome component having liposome and phosphatidylethanolamine;
(b) adding a recognizing substance component of a growth factor to the reaction vessel;
(c) buffering the reaction mixture of the liposome and recognizing substance components;
(d) adding a crosslinking reagent to the buffered reaction mixture; and
(e) allowing the buffered reaction mixture with reagent to incubate for a period of time sufficient for the modified liposome to form.

2. A process for covalently bonding a recognizing substance to a liposome comprising the steps of:
(a) providing a reaction vessel containing a liposome component having liposome and phosphatidylethanolamine;
(b) adding a recognizing substance component of growth factor to the reaction vessel;
(c) buffering the reaction mixture of the liposome and recognizing substance components;
(d) adding a crosslinking reagent to the buffered reaction mixture;
(e) allowing the buffered reaction mixture with reagent to incubate for a period of time sufficient for the covalent bonding of amine residues of the recognising substance and liposome components; and
(f) separation and removal of covalently bound recognizing substance liposome components from excess unreacted and noncovalently bound materials.

3. The process of any preceding claim wherein the liposome is selected from the group consisting of multilamellar vesicles, microemulsified liposomes and large unilamellar vesicles.

4. The process of any proceding Claim wherein the growth factor is epidermal growth factor, or urogasterone.

5. The process of any preceding claim wherein the crosslinking reagent is glutaraldehyde, a bifunctional oxirane, or ethylene glycol diglycidyl ether.

6. The process of any preceding claim wherein the recognizing substance is labelled with a fluorescent marker.

7. The process of any one of Claims 1 to 6, wherein the recognizing substance is labelled with a iodinated marker.

## Patentansprüche

1. Verfahren zum Herstellen von modifizierten Liposomen, das die folgenden Schritte aufweist:
(a) Bereitstellen eines Reaktionsgefäßes, das eine Liposomkomponente enthält, die Liposom und Phosphatidylethanolamin hat;
(b) Zufügen einer Erkennungssubstanzkomponente eines Wachstumsfaktors zu dem Reaktionsgefäß;
(c) Puffern des Reaktionsgemischs aus den Liposom- und Erkennungssubstanzkomponenten;
(d) Zufügen eines Quervernetzungsreagens zu dem gepufferten Reaktionsgemisch; und
(e) Zulassen, daß das gepufferte Reaktionsgemisch mit Reagens für einen Zeitraum inkubiert wird, der ausreicht, um das modifizierte Liposom zu bilden.

2. Verfahren zum kovalenten Binden einer Erkennungssubstanz an ein Liposom, wobei das Verfahren die folgenden Schritte aufweist:
(a) Bereitstellen eines Reaktionsgefäßes, das eine Liposomkomponente enthält, die Liposom und Phosphatidylethanolamin hat;
(b) Zufügen einer Erkennungssubstanzkomponente eines Wachstumsfaktors zu dem Reaktionsgefäß;
(c) Puffern des Reaktionsgemischs aus den Liposom- und Erkennungssubstanzkomponenten;
(d) Zufügen eines Quervernetzungsreagens zu dem gepufferten Reaktionsgemisch;
(e) Zulassen, daß das gepufferte Reaktionsgemisch mit Reagens für einen Zeitraum inkubiert wird, der für die kovalente Bindung von Aminresten der Erkennungssubstanz- und Liposomkomponenten ausreicht; und
(f) Trennen und Entfernen von kovalent gebundenen Erkennungssubstanz- und Liposomkomponenten aus nichtumgesetzten und nicht kovalent gebundenen Überschußmaterialien.

3. Verfahren nach einem der vorhergehenden Ansprüche, wobei das Liposom aus der Gruppe ausgewählt wird, die aus vielschichtigen Vesikeln, mikroemulgierten Liposomen und großen einschichtigen Vesikeln besteht.

4. Verfahren nach einem der vorhergehenden Ansprüche, wobei der Wachstumsfaktor ein Epidermis-Wachstumsfaktor oder Urogastron ist.

5. Verfahren nach einem der vorhergehenden Ansprüche, wobei das Quervernetzungsreagens Glutaraldehyd, ein bifunktionelles Oxiran oder Ethylenglykoldiglycidylether ist.

6. Verfahren nach einem der vorhergehenden Ansprüche, wobei die Erkennungssubstanz mit einem fluoreszierenden Marker gekennzeichnet wird.

7. Verfahren nach einem der Ansprüche 1 bis 6, wobei die Erkennungssubstanz mit einem iodinierten Marker gekennzeichnet wird.

## Revendications

1. Procédé permettant de produire des liposomes modifiés, comprenant les opérations consistant :
(a) à prévoir un récipient de réaction contenant un composant à base de liposome, comprenant un liposome et de la phosphatidyléthanolamine,
(b) à ajouter, dans le récipient de réaction, un composant de facteur de croissance formant une substance de reconnaissance,
(c) à tamponner le mélange de réaction du composant à base de liposome et du composant formant substance de reconnaissance,
(d) à ajouter un réactif de réticulation au mélange de réaction tamponne et
(e) à laisser incuber le mélange de réaction tamponné, comprenant le réactif, pendant une durée suffisante pour que le liposome modifié se forme.

2. Procédé permettant de lier d'une manière covalente une substance de reconnaissance à un liposome, comprenant les opérations consistant :
(a) à prévoir un récipient de réaction contenant un composant à base de liposome, comprenant un liposome et de la phosphatidyléthanolamine,
(b) à ajouter, dans le récipient de réaction, un composant de facteur de croissance formant une substance de reconnaissance,
(c) à tamponner le mélange de réaction du composant à base de liposome et du composant formant substance de reconnaissance,
(d) à ajouter un réactif de réticulation au mélange de réaction tamponné et
(e) à laisser incuber le mélange de réaction tamponné, comprenant le réactif, pendant une durée suffisante pour la liaison covalente des résidus d'amine du composant formant substance de reconnaissance et du composant à base de liposome et
(f) à séparer et extraire le composant formant substance de reconnaissance et le composant à base de liposome, liés d'une manière covalente, vis-à-vis de l'excès de matières qui n'ont pas réagit et qui ne sont pas liées d'une manière covalente.

3. Procédé selon l'une quelconque des revendications précédentes, selon lequel on choisit le liposome dans le groupe formé des vésicules multilamellaires, liposomes microémulsifiés et grosses vésicules unilamellaires.

4. Procédé selon l'une quelconque des revendications précédentes, selon lequel le facteur de croissance est le facteur de croissance épithélial ou l'urogastérone

5. Procédé selon l'une quelconque des revendications précédentes, selon lequel le réactif de réticulation est le glutaraldéhyde, un oxirane bifonctionnel ou l'éthylèneglycoldiglycidyléther.

6. Procédé selon l'une quelconque des revendications précédentes, selon lequel on marque la substance de reconnaissance au moyen d'un marqueur fluorescent.

7. Procédé selon l'une quelconque des revendications 1 à 6, selon lequel on marque la substance de reconnaissance au moyen d'un marqueur iodé.
